# EUROPEAN PATENT SPECIFICATION

(11) **EP 2 274 352 B1**
(45) Date of publication and mention of the grant of the patent: **23.05.2018**
(21) Application number: 09733829.7
(22) Date of filing: 23.04.2009
(51) Int. Cl.: C08G 18/08, C08G 18/12, C08G 18/32, C08G 18/48, C08G 18/66, C08G 18/42, C08G 18/72, A61K 8/04, A61Q 5/06

(54) **AQUEOUS DISPERSIONS OF CATIONIC FILM-FORMING POLYURETHANES**
WÄSSRIGE DISPERSION VON KATIONISCHEN FILMBILDENDEN POLYURETHANEN
DISPERSIONS AQUEUSES DE POLYURÉTHANES FILMOGÈNES CATIONIQUES

(30) Priority: 24.04.2008 IT VA20080025
(43) Date of publication of application: 19.01.2011
(73) Proprietor: Lamberti Spa, 21041 Albizzate (IT)
(72) Inventor: ALANZO, Vito, I-20022 Castano Primo (IT); COSTA, Gabriele, I-20026 Novate Milanese (IT); CONTI, Dario, I-20022 Castano Primo (IT); LI BASSI, Giuseppe, I-21026 Gavirate (IT)
(86) International application number: PCT/EP2009/054874
(87) International publication number: WO 2009/130269

(56) References cited:
- EP-A- 0 554 747
- FR-A- 2 815 350
- US-A- 4 293 474
- US-B1- 6 313 335

## Description

### FIELD OF THE INVENTION

The present invention relates to aqueous dispersions of film-forming cationic polyurethanes and to their use in the preparation of cosmetics and personal care products.

According to one of the fundamental aspect of the present invention the procedure for the preparation of the above aqueous dispersions is described.

### STATE OF THE ART

Aqueous dispersions of cationic polyurethanes which are capable of producing films (film-forming polyurethanes) are known; their preparation and use in many industrial fields, such as in the field of cosmetics, textiles, leather and paper, is described in several patents.

By way of example, US 3,388,087 describes the preparation of aqueous dispersions of quaternized polyurethanes which are useful for the preparation of flexible films that are useful in the cosmetic industry as ingredients of hair fixative lacquers.

US 3,480,592 describes the preparation of aqueous dispersions of polyurethanes containing salified tertiary amine groups; the films obtained from these polyurethanes have applications that are similar to those of US 3,388,087.

More recently, US 6,335,003 reports the preparation of polyurethanes containing salified tertiary amine groups; the polyurethanes of US 6,335,003 are different from the polyurethanes of US 3,480,592 because of their high amine number.

US 2004/0052753 describes elastic polyurethanes containing salified tertiary amine groups and their use in cosmetic compositions.

At least hypothetically, aqueous dispersions of cationic film-forming polyurethanes can constitute a valid alternative to the commonly used ureaformaldehyde resins, the use of which has become critical due to more and more strict regulatory issues on formaldehyde residues in products for personal use (textiles included).

Nonetheless, relatively few products of these kinds are available on the market.

The main problem which is encountered in the preparation of aqueous dispersions of film-forming cationic polyurethanes is the balance of their components, that shall guarantee, at the same time, good dispersibility and stability in water, and good elasticity.

The Applicant has now found that remarkably stable aqueous dispersions of film-forming cationic polyurethanes can be obtained by using in their preparation aliphatic diisocyanates and methanesulfonic acid as the salifying agent of the tertiary amine groups of the polymer.

The improved stability of the aqueous dispersions, beside providing positive effects on their storability, also advantageously increases the transparency and gloss of the films obtained there from.

According to the Applicant's knowledge, the stabilizing effect of the combination of aliphatic diisocyanate with methanesulfonic acid is not known from the literature, even if methanesulfonic acid is cited in US 3,480,592 (col. 4, I. 35 - col. 5, I. 60) among a long list of utilizable compounds as a possible salifying agent for cationic polyurethanes.

### DESCRIPTION

Accordingly, the fundamental object of the present invention is an aqueous dispersion of film-forming cationic polyurethanes obtained by: A) reacting one or more aliphatic diisocyanates with an aliphatic tertiary amine having two hydroxyl groups that react with -NCO groups and a linear polyol of the polyol polyether or polyol polyester type having molecular weight from 500 to 5,000, to obtain a prepolymer having free isocyanate groups; B) salifying with methanesulfonic acid the amine groups of the prepolymer; C) extending the thus obtained cationic prepolymer with water or amines, and wherein in step A), the molar ratio between aliphatic diisocyanates and compounds having two reactive hydroxyl groups (linear polyol and aliphatic tertiary amine) is from 1.2 to 2.2, and preferably from 1.3 to 1.8. The cationic polyurethane of the present invention has molecular weight from 2,000 and 50,000.

The diisocyanates that can be used according to the present invention are aliphatic; with the term "aliphatic" we mean both aliphatic and cycloaliphatic; preferably the diisocyanates are chosen among 4,4'-dicyclohexyl methane-diisocyanate, 1-isocyanate, 3-isocyanate-methyl-3,5,5-trimethylcyclohexane (or isophorone diisocyanate), hexamethylene diisocyanate and mixtures thereof.

The aliphatic amine having two hydroxyl groups that react with the -NCO group is preferably 3-(diethylamino)-1,2-propanediol or an alkyl diethanol amine; among the alkyl diethanol amines, methyl-, ethyl, isopropyl-, n-butyl-, t-butyl-, cyclohexyl-, n-hexyl- diethanolamine, and mixtures thereof are particularly useful.

The amount of amine which is bound in the polyurethane is measured in milliequivalent of amine and is calculated as the percentage of the ratio of amine millimoles on grams of dry polyurethane.

In the present invention the milliequivalents of amine in the polyurethanes range from 20 to 150, preferably from 30 to 90.

The linear polyols that may be used for the invention have molecular weight from 500 to 5,000, preferably from 800 to 3,000 and they are preferably hydroxyl terminated polyether or polyester diols.

Examples of polyol polyester that may be used according to the present invention are the products obtained from the polycondensation of acids or anhydrides, having from 4 to 9 carbon atoms and preferably of the aliphatic kind, with aliphatic diols having from 2 to 8 carbon atoms.

Among the preferred polyol polyester we cite polyadipate from 1,4-butandiol and ethylene glycol, polyadipate from 1,6-hexanediol and neopentylglycol, polyadipate phthalate from 1,6 hexanediol, polycaprolactones and polycarbonate diols from 1,6-hexanediol and 1,4-butanediol.

Polyadipate from 1,4-butandiol and ethylene glycol, polyadipate from 1,6-hexanediol and neopentylglycol, polyadipate phthalate from 1,6 hexanediol are particularly preferred.

Examples of polyol polyethers that may be used are polyethylene glycol, polytetrahydrofuran, and mixtures thereof; the latter is preferred for the realization of the present invention.

Mixtures of different linear polyols may be used in step A).

In the most common embodiments, step A) is carried out without any solvent, but, preferably, at the end of step A) and before conducting the salification of step B) a non reactive organic solvent, by way of example N-methylpyrrolidone or acetone, is added.

The salification is preferably carried out at room temperature by using methanesulfonic acid in one of its more common commercially available forms, that is as a 70% w/w aqueous solution.

The salification step is made by adding methanesulfonic acid in molar amounts ranging from 60 to 120%, more preferably from 95 to 105%, the percentage being referred to the moles of tertiary amine.

The extension of the cationic polyurethane obtained after step B) is preferably made by reaction with water at temperature of 20-60°C.

Typically, in the final aqueous dispersion the content of film-forming cationic polyurethane is from 20 to 35% by weight.

This range of concentrations is particularly appreciable for industrial use because it corresponds to sufficiently concentrated dispersions that avoid the useless transportation of large amounts of water and; at the same time, the viscosity of the dispersions in this range of concentrations allows their easy handling.

Moreover, according to here described invention, it is also possible to obtain cationic dispersions that are free from solvents and have low content of volatile organic compounds.

The aqueous dispersions of film-forming polyurethanes of the present invention are advantageously used in the preparation of cosmetic products, such as make up products (mascaras, long lasting lipsticks), hair lacquers, nail varnishes, hair gels, waterproof sunscreen creams.

The cationic nature of the dispersions of the present application makes them particularly affine to keratin and therefore to hair; they are advantageously employed in products for the treatment and care of hair.

Beside the cosmetic field, the dispersions of the invention are useful in leather finishing, in textile sizing and finishing, in the coating of paper, wood and plastic, as ant felting agents for wool.

In the following examples the preparation of aqueous dispersions according to the present invention is reported (Ex. I-III, V) and of comparative dispersions (Ex. IV, VI-VIII); the reported percentages are by weight.

In an applicative example the preparation of a hair lacquer from an aqueous dispersion according to the invention is described.

### EXAMPLES

In the examples the following materials were used.
Polyol 1 = polyadipate from 1,4-butanediol and ethylene glycol having molecular weight 830 g/mol.
Polyol 2 = polytetramethylene ether glycol having molecular weight 1000 g/mol
Amine 1 = methyldietanol amine (MW 119.16 g/mol)
Amine 2 = 3-(diethylamino)-1,2-propanediol (MW 147.22 g/mol)
Acid 1 = methanesulfonic acid (MW 96.11 g/mol), 70% aqueous solution
Acid 2 = p-toluenesulfonic acid (MW 172.2 g/mol), 20% aqueous solution
Acid 3 = phosphoric acid (MW 98.00 g/mol), 75% aqueous solution
Acid 4 = formic acid (MW 46.03 g/mol), 85% aqueous solution
Diisocyanate 1 = 4-4'-methylene-bis-(4-cyclohexyl isocyanate), MW 262.35 g/mol (Desmodur W, Bayer).
Diisocyanate 2 = Hexamethylene diisocyanate, MW 168.19 (Desmodur I, Bayer).
Diisocyanate 3 = Toluene diisocyanate, MW 174.16 (Desmodur T80, Bayer)

### EXAMPLE I

Preparation of a cationic polyurethane dispersion according to the invention. A reaction vessel, equipped with internal thermometer, stirrer and cooler, was filled, under nitrogen atmosphere and at room temperature, with 160.21 g (160 mmol) of Polyol 2 (fed at 40°C), 17.87 g (150 mmol) of Amine 1 and 0.048 g of benzoyl chloride. The mixture was heated to 40°C and stirred for 30 minutes. 121.91 g (465 mmol) of Diisocyanate 1 were added under stirring to the homogeneous mixture which was then maintained at 60°C for 30 minutes. The reaction temperature was brought to 90°C until the titrimetric determination of the free -NCO groups still present gave a calculated value of 4.35% by weight (value determined in this example as well as in other examples according to the standard method ASTM D2572).

Once obtained the above NCO value, the prepolymer was cooled to 60°C adding at the same time 105 g of acetone. At about 40°C, 20.59 g (150 mmol) of Acid 1 were added under stirring and after 10 minutes, at temperature of 40°C, 679.3 g of demineralized water were added. Afterwards acetone was distilled off under vacuum while keeping the cationic waterborne polyurethane dispersion under stirring to obtain a stable translucent product with 30% solid content.

At the end of the distillation process the -NCO peak in the IR spectrum at 2240 cm-1 had disappeared.

The obtained cationic aqueous polyurethane dispersion is stable for more than 6 months.

### EXAMPLES II - VIII

The procedure of Example I was followed while changing the ratios and raw materials as reported in Table 1a (examples according to the invention) and in Table 2a (comparative examples).

### Tables 1b and 2b report the peculiarities of the polyurethane dispersions characteristics.

**Table 1a (amount of raw materials)**

| | Example I | | Example II | | Example III | | Example V | |
|---|---|---|---|---|---|---|---|---|
| | g | mmol | g | mmol | g | mmol | g | mmol |
| POLYOL 1 | - | | 168.34 | 203 | 173.57 | 209 | - | - |
| POLYOL 2 | 160.2 | 160 | - | - | - | - | 177 | 177 |
| AMINE 1 | 17.87 | 150 | 17.87 | 150 | - | - | 19.66 | 165 |
| AMINE 2 | - | | - | - | 22,08 | 150 | - | - |
| DIISOCYANATE 1 | 121.91 | 465 | 44.45 | 169 | 40.77 | 155 | 40.25 | 153 |
| DIISOCYANATE 2 | - | | 69.33 | 412 | 63.58 | 378 | 62.77 | 373 |
| ACID 1 | 20.59 | 150 | 20.59 | 150 | 20.59 | 150 | 22.65 | 165 |
| DEMINERALIZED WATER | 679.25 | | 679.25 | | 679.0 | | 670 | |
| ACETONE | 105 | | 110 | | 110 | | 110 | |

**Table 1b - Dispersions characteristics**

| | Example I | Example II | Example III | Example V |
|---|---|---|---|---|
| % NCO PREPOLYMER | 4.35% | 4.94% | 4.53% | 3.84% |
| SOLID CONTENT | 30% | 30% | 30% | 30% |
| SHELF LIFE | > 6 months | > 6 months | > 6 months | > 6 months |

**Table 2a (amounts of raw materials)**

| | Example IV* | | Example VI* | | Example VII* | | Example VIII* | |
|---|---|---|---|---|---|---|---|---|
| | g | mmol | g | mmol | g | mmol | g | mmol |
| POLYOL 1 | - | - | - | - | - | - | - | - |
| POLYOL 2 | 160.21 | 160 | 227.5 | 227 | 175.51 | 175 | 161.81 | 162 |
| AMINE 1 | 17.87 | 150 | 16.68 | 140 | 18.3 | 153 | 15.01 | 126 |
| DIISOCYANATE 1 | 121.91 | 465 | - | - | 41.46 | 158 | - | - |
| DIISOCYANATE 2 | - | - | 83.35 | 495 | 64.72 | 384 | - | - |
| DIISOCYANATE 3 | - | - | - | - | - | - | 75.11 | 431 |
| ACID 1 | - | - | - | - | - | - | 17.29 | 126 |
| ACID 2 | 129.0 | 150 | - | - | - | - | - | - |
| ACID 3 | - | - | - | - | 20.00 | 153 | - | - |
| ACID 4 | - | - | 7.49 | 138 | - | - | - | - |
| DEMINERALIZED WATER | 545.0 | | 677.0 | | 680.0 | | 573 | |
| ACETONE | 105 | | 105 | | 110 | | 125 | |

| | | | | | | | | |
|---|---|---|---|---|---|---|---|---|
| *comparative | | | | | | | | |

**Table 2b - Characteristics of the Dispersions**

| | Example IV* | Example VI* | Example VII* | Example VIII* |
|---|---|---|---|---|
| % NCO PREPOLYMER | 4.35% | 3.3% | 4.5% | 4.8% |
| SOLID CONTENT | N.D. | 33% | 30% | N.D. |
| SHELF LIFE | 30' | 60' | 80' | 0** |

| | | | | |
|---|---|---|---|---|
| * comparative **the dispersion separates during its preparation process. | | | | |

### APPLICATION TEST.

Two standard formulations were prepared to evaluate the performances of the product obtained from Example I in comparison with a standard film forming polymer used in cosmetic for hair styling. The comparative polymer used was a polyvinilpirrolidone/vinylacetate (CTFA name: VP/VA Copolymer, LUVISKOL® VA64 P from BASF Aktiengesellschaft)

The formulations have the following composition (a.c. = active content).
Formulation 1:

| | |
|---|---|
| Dispersion obtained by Example 1 (a.c. 30% w/w) | 6% |
| Water/Ethanol Mixture 1/2 | to 100% |

Formulation 2:

| | |
|---|---|
| LUVISKOL VA64 P (a.c. >95% w/w) | 1.8% |
| Water/Ethanol Mixture 1/2 | to 100% |

The two film forming polymers, the polyurethane from Example I and Luviskol VA64 P, are completely dissolved into the Water/Ethanol mixture and Formulation 1 and 2 are transferred into spray containers.

To test their film forming effectiveness, 1 g of each Formulation is sprayed on a lock and dried for 1 hr. A panel test carried out on 10 persons showed that the finishing obtained with Formulation 1 is softer.

To test the curl retention, 1 g of each Formulation is sprayed onto a lock of medium/long length and set with a curler laying for 1 hr. A panel test carried out on 10 persons showed that the finishing obtained with Formulation 1 is more natural and the lock is better styled.

It is therefore pointed out that the polyurethane dispersion obtained from Example I shows good finishing performance and set on hair.

## Claims

1. Aqueous dispersion of film-forming cationic polyurethanes obtained by:
A) reacting one or more aliphatic diisocyanates with an aliphatic tertiary amine having two hydroxyl groups that react with -NCO groups and a linear polyol of the polyol polyether or polyol polyester type having molecular weight from 500 to 5,000, to obtain a prepolymer having free isocyanate groups; B) salifying with methanesulfonic acid the amine groups of the prepolymer; C) extending the thus obtained cationic prepolymer with water or amines, wherein in step A), the molar ratio between aliphatic diisocyanates and the sum of linear polyol and aliphatic tertiary amine is from 1.2 to 2.2 and the linear polyols are hydroxyl terminated polyether or polyester diols.

2. Aqueous dispersion of film-forming cationic polyurethanes according to claim 1, wherein the diisocyanates are chosen among 4,4'-dicyclohexyl methane-diisocyanate, 1-isocyanate, 3-isocyanate-methyl-3,5,5-trimethylcyclohexane (or isophorone diisocyanate), hexamethylene diisocyanate and mixtures thereof.

3. Aqueous dispersion of film-forming cationic polyurethanes according to claim 2, wherein the aliphatic tertiary amine having two hydroxyl groups that react with the -NCO group is preferably 3-(diethylamino)-1,2-propanediol or an alkyl diethanol amine.

4. Aqueous dispersion of film-forming cationic polyurethanes according to claim 3, wherein the alkyl diethanol amine is chosen among methyl-, ethyl, isopropyl-, n-butyl-, t-butyl-, cyclohexyl-, n-hexyl-diethanolamine, and mixtures thereof.

5. Aqueous dispersion of film-forming cationic polyurethanes according to claim 3, wherein the polyol is polyadipate from 1,4-butandiol and ethylene glycol, polyadipate from 1,6-hexanediol and neopentylglycol, or polyadipate phthalate from 1,6 hexanediol.

6. Aqueous dispersion of film-forming cationic polyurethanes according to claim 3, wherein the polyol is polytertrahydrofuran.

7. Aqueous dispersion of film-forming cationic polyurethanes according to any of the preceding claims wherein the salification step is made by adding methanesulfonic acid in molar amounts ranging from 60 to 120%, more preferably from 95 to 105%, the percentage being referred to the moles of tertiary amine.

8. Aqueous dispersion of film-forming cationic polyurethanes according to claim 7, wherein the extension of the cationic polyurethane obtained after step B) is preferably made by reaction with water at temperature of 20-60°C.

9. Aqueous dispersion of film-forming cationic polyurethanes according to any of the preceding claims wherein the content of film-forming cationic polyurethanes is from 20 to 35% by weight.

10. Cosmetic compositions comprising an aqueous dispersion of film-forming cationic polyurethanes according to any of claims from 1 to 9.

11. Use of the aqueous dispersions of film-forming cationic polyurethanes according to any of claims from 1 to 9 in the preparation of cosmetic compositions, in leather finishing, in textile sizing and finishing, in the coating of paper, wood and plastics and as anti felting agents for wool.

## Patentansprüche

1. Wässrige Dispersion von filmbildenden kationischen Polyurethanen, erhalten durch:
A) Umsetzung eines oder mehrerer aliphatischer Diisocyanate mit einem aliphatischen tertiären Amin mit zwei Hydroxylgruppen, die mit -NCO-Gruppen reagieren, und einem linearen Polyol vom Polyolpolyether- oder Polyolpolyester-Typ mit einem Molekulargewicht von 500 bis 5000, um ein Präpolymer zu erhalten, das freie Isocyanatgruppen aufweist;
B) Salzbildung der Amingruppen des Präpolymers mit Methansulfonsäure;
C) Verlängerung des so erhaltenen kationischen Präpolymers mit Wasser oder Aminen,
wobei in Schritt A) das Molverhältnis zwischen aliphatischen Diisocyanaten und der Summe von linearem Polyol und aliphatischem tertiärem Amin 1,2 bis 2,2 beträgt und die linearen Polyole Hydroxyl-terminierte Polyether- oder Polyesterdiole sind.

2. Wässrige Dispersion von filmbildenden kationischen Polyurethanen nach Anspruch 1, wobei die Diisocyanate aus 4,4'-Dicyclohexylmethandiisocyanat, 1-Isocyanat-3-isocyanatmethyl-3,5,5-trimethylcyclohexan (oder Isophorondiisocyanat), Hexamethylendiisocyanat und Mischungen davon ausgewählt sind.

3. Wässrige Dispersion von filmbildenden kationischen Polyurethanen nach Anspruch 2, wobei das aliphatische tertiäre Amin mit zwei Hydroxylgruppen, die mit der -NCO-Gruppe reagieren, vorzugsweise 3-(Diethylamino)-1,2-propandiol oder ein Alkyldiethanolamin ist.

4. Wässrige Dispersion von filmbildenden kationischen Polyurethanen nach Anspruch 3, wobei das Alkyldiethanolamin ausgewählt ist aus Methyl-, Ethyl-, Isopropyl-, n-Butyl-, t-Butyl-, Cyclohexyl-, n-Hexyl-diethanolamin und Mischungen davon.

5. Wässrige Dispersion von filmbildenden kationischen Polyurethanen nach Anspruch 3, wobei das Polyol Polyadipat von 1,4-Butandiol und Ethylenglykol, Polyadipat von 1,6-Hexandiol und Neopentylglykol oder Polyadipatphthalat von 1,6-Hexandiol ist.

6. Wässrige Dispersion von filmbildenden kationischen Polyurethanen nach Anspruch 3, wobei das Polyol Polytetrahydrofuran ist.

7. Wässrige Dispersion von filmbildenden kationischen Polyurethanen nach einem der vorhergehenden Ansprüche, wobei der Salzbildungsschritt durch Zugabe von Methansulfonsäure in molaren Mengen im Bereich von 60 bis 120%, bevorzugter von 95 bis 105%, erfolgt, wobei die Prozentangabe auf die Mole von tertiärem Amin bezogen ist.

8. Wässrige Dispersion von filmbildenden kationischen Polyurethanen nach Anspruch 7, wobei die Verlängerung des nach Schritt B) erhaltenen kationischen Polyurethans vorzugsweise durch Reaktion mit Wasser bei einer Temperatur von 20 bis 60 °C erfolgt.

9. Wässrige Dispersion von filmbildenden kationischen Polyurethanen nach einem der vorhergehenden Ansprüche, wobei der Gehalt an filmbildenden kationischen Polyurethanen 20 bis 35 Gew.-% beträgt.

10. Kosmetische Zusammensetzungen, die eine wässrige Dispersion von filmbildenden kationischen Polyurethanen nach einem der Ansprüche 1 bis 9 umfassen.

11. Verwendung der wässrigen Dispersionen von filmbildenden kationischen Polyurethanen gemäß einem der Ansprüche 1 bis 9 bei der Herstellung von kosmetischen Zusammensetzungen, bei der Lederzurichtung, bei der Textilschlichte und Textilveredelung, bei der Beschichtung von Papier, Holz und Kunststoffen und als Antifilzmittel für Wolle.

## Revendications

1. Dispersion aqueuse de polyuréthanes cationiques filmogènes obtenue par : A) réaction d'un ou plusieurs diisocyanates aliphatiques avec une amine tertiaire aliphatique ayant deux groupes hydroxyle qui réagissent avec les groupes -NCO et un polyol linéaire de type polyol-polyéther ou polyol-polyester ayant une masse moléculaire de 500 à 5 000, pour que soit obtenu un prépolymère ayant des groupes isocyanate libres ; B) salification avec de l'acide méthanesulfonique des groupes amine du prépolymère ; C) allongement du prépolymère cationique ainsi obtenu avec de l'eau ou des amines, dans laquelle, dans l'étape A), le rapport molaire entre les diisocyanates aliphatiques et la somme du polyol linéaire et de l'amine tertiaire aliphatique est de 1,2 à 2,2 et les polyols linéaires sont des polyéther- ou polyester-diols à terminaison hydroxyle.

2. Dispersion aqueuse de polyuréthanes cationiques filmogènes selon la revendication 1, dans laquelle les diisocyanates sont choisis parmi le diisocyanate de 4,4'-dicyclohexylméthane, le 1-isocyanato, 3-isocyanato-méthyl-3,5,5-triméthylcyclohexane (ou diisocyanate d'isophorone), le diisocyanate d'hexaméthylène, et leurs mélanges.

3. Dispersion aqueuse de polyuréthanes cationiques filmogènes selon la revendication 2, dans laquelle l'amine tertiaire aliphatique ayant deux groupes hydroxyle qui réagissent avec le groupe -NCO est de préférence le 3-(diéthylamino)-1,2-propanediol ou une alkyldiéthanolamine.

4. Dispersion aqueuse de polyuréthanes cationiques filmogènes selon la revendication 3, dans laquelle l'alkyldiéthanolamine est choisie parmi les méthyl-, éthyl-, isopropyl-, n-butyl-, t-butyl-, cyclohexyl-, n-hexyl-diéthanolamines, et leurs mélanges.

5. Dispersion aqueuse de polyuréthanes cationiques filmogènes selon la revendication 3, dans laquelle le polyol est un polyadipate dérivé de 1,4-butanediol et d'éthylèneglycol, un polyadipate dérivé de 1,6-hexanediol et de néopentylglycol, ou un polyadipate-phtalate dérivé de 1,6-hexanediol.

6. Dispersion aqueuse de polyuréthanes cationiques filmogènes selon la revendication 3, dans laquelle le polyol est le polytétrahydrofurane.

7. Dispersion aqueuse de polyuréthanes cationiques filmogènes selon l'une quelconque des revendications précédentes, dans laquelle l'étape de salification est mise en oeuvre par addition d'acide méthanesulfonique en une quantité molaire située dans la plage allant de 60 à 120 %, mieux encore de 95 à 105 %, le pourcentage se rapportant aux moles de l'amine tertiaire.

8. Dispersion aqueuse de polyuréthanes cationiques filmogènes selon la revendication 7, dans laquelle l'allongement du polyuréthane cationique obtenu après l'étape B) est de préférence effectué par réaction avec de l'eau à une température de 20 à 60 °C.

9. Dispersion aqueuse de polyuréthanes cationiques filmogènes selon l'une quelconque des revendications précédentes, dans laquelle la teneur en les polyuréthanes cationiques filmogènes est de 20 à 35 % en poids.

10. Compositions cosmétiques comprenant une dispersion aqueuse de polyuréthanes cationiques filmogènes selon l'une quelconque des revendications 1 à 9.

11. Utilisation de dispersions aqueuses de polyuréthanes cationiques filmogènes selon l'une quelconque des revendications 1 à 9 dans la préparation de compositions cosmétiques, dans le vernissage du cuir, dans l'ensimage et le finissage de textiles, dans le couchage du papier, dans le revêtement du bois et des matières plastiques, et en tant qu'agents anti-feutrage pour la laine.
